# EUROPEAN PATENT APPLICATION

(11) **EP 3 127 557 A1**
(43) Date of publication of application: **08.02.2017**
(21) Application number: 16020296.6
(22) Date of filing: 05.08.2016
(51) Int. Cl.: A61K 47/10, A61K 9/06, A61K 31/00, A61P 35/00, A61K 41/00

(54) **A VEHICLE FOR TOPICAL PHOTODYNAMIC THERAPY WITH DELTA-AMINOLEVULINIC ACID IN DERMATOLOGIC APPLICATIONS, AND METHOD FOR PREPARING THE VEHICLE**

(30) Priority: 05.08.2015 IT UB20152862
(71) Applicant: TERES S.r.l., 20121 Milan (IT); Fraschini, Franco Luigi, 20123 Milano (IT); Fumagalli, Marco Carlo, 20154 Milano (IT); Alpha Strumenti S.r.l., 20066 Melzo (MI) (IT)
(72) Inventor: Ceschel, Giancarlo Antonio Renato, I-20121 Milano (IT)
(74) Representative: Saporiti, Emilio Luigi

(57) **Abstract**

An ALA-gel vehicle for favoring an absorption and conveying of an active principle for a topic use in particular in a photodynamic therapy with delta-aminolevulinic acid in dermatologic applications, said vehicle comprising a clear aqueous solution jelling at a temperature from 20 to 37°C, preferably from 25 to 27°C, of the following components: poloxamer 407 (Lutrol 127) as a vehicle-gel either per se or in a mixture with one or more of the poloxamers having the following structural formula: 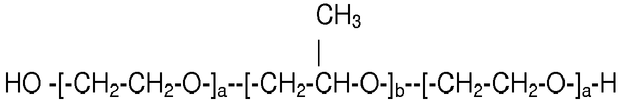 said vehicle comprising moreover potassium sorbate and sodium benzoate as preserving agents, 5-amminolevunic chloridrate as a precursor agent for providing a target photodynamic reaction, and purified water.

## Description

### BACKGROUND OF THE INVENTION

The present invention relates, according to one aspect thereof, to a vehicle, in particular for a photodynamic therapy with delta-aminolevulinic acid (ALA-PDT) in a dermatologic field.

According to a further aspect thereof, the invention relates to a method for preparing the vehicle in a form of a gel.

As is known, by the term "vehicle" is generally indicated an inactive substance used for presenting in the most suitable manner active substances mixed therewith, said term being a synonym, in the pharmaceutical filed, of "excipient", that is a substance devoid of any pharmacologic activity, to be mixed with an active substance for use in a desired form, thereby facilitating, for example, the administering thereof.

Also known is the fact that by "topic photodynamic therapy with ALA (ALA-PDT) in the dermatologic field", [DOUG-98] is meant an application of a topic preparation containing delta-aminolevulinic acid (ALA) on a skin area to be treated; followed by an incubation period (frequently by an application of an occlusive bandage) and then by a light source radiation to activate the photosensitizing agent.

While ALA per se is not a photosensitizing agent, a product of its metabolic transformation: Protoporphyrin-9 (PpIX-9) is an efficient photosensitizing agent allowing a desired therapeutic effect to be achieved.

The PpIX-9 is activated by a light source (such as a LED array) with a peak emission at 630 nm; with a corresponding tissue penetration of the light of about 2 mm.

The conversion rate of ALA into PpIX-9 is enhanced in the tissue affected by a lesion to be treated; by suitably metering an incubating time, a PpIX-9 concentration gradient allowing to perform a selective treatment is achieved.

Said PpIX-9 has a strong fluorescence in red if it is excited by a violet source (lambda = 405 nm) [FLUO-02]: this allows both to detect the lesion edges and to evaluate "in vivo" the PpIX-09 concentration in the tissues.

The most part of the available literature [LANC-04] [GUID-07] relates to strictly clinic (oncologic) applications; recently [PHAGE-08] dermo-aesthetic applications have been introduced, for example in treating skin photo-ageing and acne phenomena.

At present, a factor greatly limiting a diffusion of the above therapy is the excessively long duration of the incubating times and a discomfort caused by the occlusive bandage (in acne and photo-rejuvenation the face constitutes the skin area which is involved in most of the cases).

At present, ALA is topically prepared in an extemporaneous manner by mixing ALA (a crystalline powder which is stable at room temperature) with different vehicles:
- ointment M
- A. cream base
- a PEG ointment (a mixture of PEG at different densities).

The ALA concentrations range from 20% to 10%.

The mixture thus obtained is stable for 0-15 days if preserved at a temperature of 4°C.

The incubating times range from 2 to 4 hours and an occlusion is always necessary.

### SUMMARY OF THE INVENTION

The aim of the present invention is to provide a novel ALA-based vehicle, which will be hereinafter also called "ALA-gel", which is both very stable and effective even at a reduced concentration of said ALA.

Within the scope of the above mentioned aim, a main object of the present invention is to provide a novel "ALA-gel" vehicle, which has a reduced incubating time and does not require any occlusion step.

Another object of the present invention is to provide such a vehicle in a form of a gel, or ALA-gel, allowing any active principle compatible therewith to remain in contact with the skin tissue for enough time to provide a suitable therapeutic effect even if the active principle tends to render said the ALA-gel vehicle unstable for its intended topic application, and this without a need of increasing the dose or amount of the active principle contained in the topical use preparation.

Yet another object of the present invention is to provide such a vehicle, specifically designed for the above mentioned dermatologic applications, adapted to allow a long duration releasing of the active principle, without any risk of possible local negative reactions.

According to one aspect of the present invention, the above mentioned aim and objects, as well as yet other objects which will become more apparent hereinafter, are achieved by an ALA-gel vehicle and a method for preparing it according to the accompanying claims.

### BRIEF DESCRIPTION OF THE DRAWINGS

Further characteristics and advantages of the ALA-gel vehicle of the present invention and the preparing method therefor will become more apparent hereinafter from the following detailed disclosure of some preferred embodiments thereof, with reference to the accompanying drawings, where:

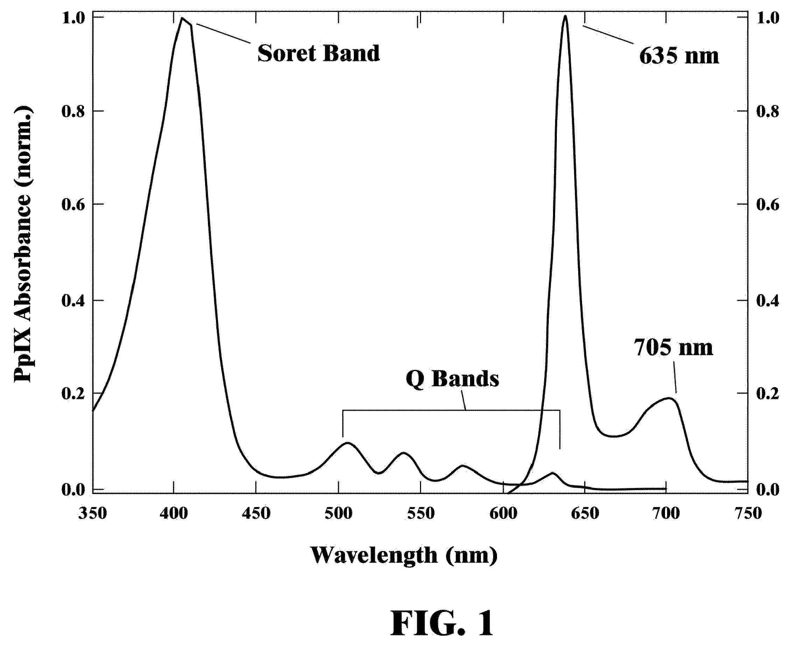

Figure 1 shows a PpIX absorbance diagram related to the wavelength, which is useful to understand the inventive ALA-gel vehicle efficiency in topic dermatologic applications according to the present invention; and

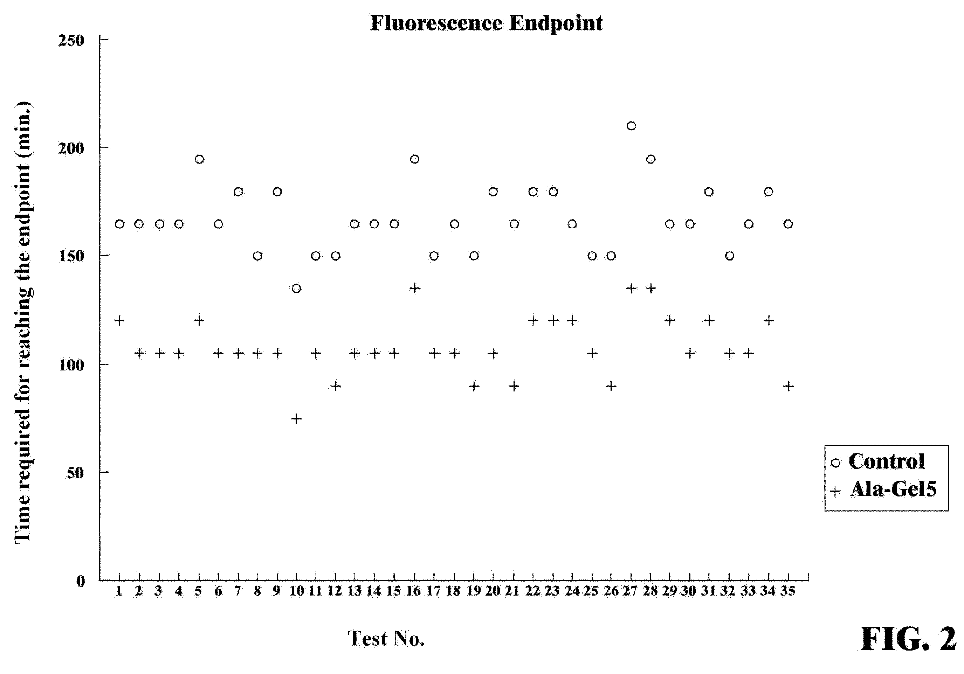

Figure 2 shows a further diagram illustrating the time period required to achieve the fluorescence endpoint related to the number of the test performed, with an application of a control vehicle and the inventive ALA-gel vehicle, this diagram being also useful to understand the inventive ALA-gel vehicle efficiency in topic dermatologic applications provided according to the present invention.

### DESCRIPTION OF THE PREFERRED EMBODIMENTS

Before discussing in a detailed manner the ALA-gel vehicle and its efficiency in the target topic dermatologic applications and its preparing method, according to the present invention, it should be pointed out that the term "gel", as herein disclosed, means a colloidal mass or system, with an aqueous base, having a jelly aspect.

Moreover, the expression "thermally reversible gel" means a system made of aqueous (co)polymeric solutions, including as (co)polymer a poloxamer, adapted to be jelled in a reversible manner in response to a gel temperature variation. Typically, such a jellification or jelling of said aqueous polymeric solution may occur in situ, for example because of a temperature increase occurring as said (co)polymeric solution contacts a body tissue.

According to embodiments of the invention, the inventive vehicle has a liquid status at a temperature lower than room temperature and is in a form of a gel at a temperature near the human body temperature.

For example, the inventive vehicle could have a liquid status at a temperature of 5-15°C, and a gel status as it is exposed to a temperature near the outer body temperature (about 37°C).

In disclosing the present invention, the term "poloxamer" will mean block copolymers (that is copolymers including individual monomers forming blocks of different length) being essentially formed by three blocks: a central block (a hydrophobic one) of polypropylene oxide (PPO) to the ends of which are coupled two (hydrophilic) blocks made of polyethylene oxide (PEO).

Typically, poloxamers are derived from a sequential polymerization at first of propylene oxide (PO) and then of ethylene oxide (EO) to a propylene glycol molecule. These operating stages, generally oxyalkylation stages, are performed in a presence of one or more alkali catalyzers, for example potassium and sodium hydroxide, which are then neutralized and removed from the end product.

The inventive poloxamers are represented by the following structural formula:

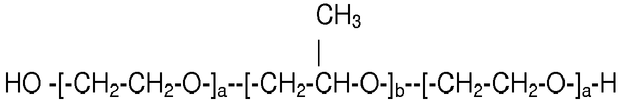

The poloxamers used in carrying out the present invention are copolymers whose viscosity increases (that is they are "thickened") in an aqueous solution so as to form thermally reversible gels whose rheologic properties change depending on their concentration and molecular weight.

Generally, the diluted poloxamer aqueous solutions will have a Newton flow.

Only by way of an example, at a rate higher than 20% by weight, said aqueous solutions perform a transition to a plastic flow, thereby substantially modifying their viscosity and flow properties.

The concentration and temperature influence on the above mentioned poloxamer gel aqueous solutions are disclosed, for example, by Lenaerts, Vincent et al. in Int. J. Pharm. (1987) 39, (1-2) 121-7, whose contents are herein fully incorporated by reference.

Poloxamers that can be used in performing the present invention may have a polymeric chain of a variable length.

In the preferred embodiment of the invention, the poloxamer which has been found as the most advantageous one in performing the present invention is Poloxamer 407 (Lutrol F 127 Prill) and CAS-No. 900-11-6 and mixtures thereof (available from BASF CHEMICAL COMPANY).

The Applicant has surprisingly found that the use of the above mentioned specific poloxamer allows to optimize the active principle dose-effect ratio and to extend in a suitable manner its releasing time, thereby facilitating a very quick healing of the tissues requiring the subject treatment.

This effect is neither disclosed nor suggested by any BASF technical specification, that is by the maker of the above mentioned poloxamer.

In fact, even though specifications of this product show that Lutrol 127 is mainly used as a thickening and gel-forming agent or as an emulsifying agent and cream or liquid emulsion consistency improving agent or as a solubilizing agent for some active substances for formulating active products having a reduced solubility due to neutralization, its applications, as disclosed and suggested by BASF, are limited to a use as a stabilizing product, in particular in orally administered suspensions, or in toothpaste and mouthwash compositions.

According to a main aspect thereof, the invention provides an ALA-gel vehicle to facilitate an absorbing and conveying of any desired active principle for topical use, in particular for a topic photodynamic therapy by delta-aminolevulinic acid in a dermatologic application, which vehicle comprises a clear aqueous solution which is jelled at a temperature from 20 to 37°C, preferably from 25 to 27°C, of the following components: poloxamer 407 (Lutrol 127) as a vehicle, either per se or in a mixture with one or more of other poloxamers having the following structural formula: 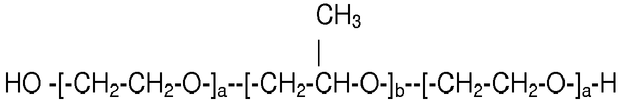 and with potassium sorbate and sodium benzoate as preserving agents; with 5-aminolevulinic chloridrate, as a precursor for developing a desired photodynamic reaction; and purified water.

According to a second aspect thereof, the present invention provides an ALA-gel vehicle having the following formulation, in weight percent: poloxamer 407 (Lutrol 127) from 1 to 40%, preferably 17%; potassium sorbate 0.20%; sodium benzoate 0.5%; 5-aminolevulinic chloridrate from 0.1 to 30%, preferably 1.00%, and purified water q.s. to 100%.

According to a third aspect thereof, the present invention provides an ALA-gel vehicle which may also be preserved separately from the other components, and in which said vehicle is extemporaneously added to any desired active principle, before use, by adding said ALA-gel vehicle even to other optional components of the topic use pharmaceutical preparation, including the desired active principle whose releasing must be extended by the inventive vehicle.

According to a fourth aspect thereof, the present invention provides a method for preparing an ALA-gel vehicle according to any of the above three aspects, which method comprises at least the steps of: cooling a preset amount of purified water; dissolving under stirring potassium sorbate or sodium benzoate in said purified water; dispersing 5-aminolevulinic chloridrate and poloxamer 407 (Lutrol 127) and further stirring the dispersion thus obtained; cooling said stirred dispersion up to a full dissolving of said poloxamer 407 (Lutrol 127), thereby providing a clear solution jelling in a temperature range of substantially 25-27°C.

According to a fifth aspect of the present invention, in the method according to the fourth aspect, said purified water preset amount is cooled to +5°C.

According to a sixth aspect of the present invention, in the method according to the fourth aspect, said dispersion is further stirred for 10-15 minutes.

According to a seventh aspect of the present invention, in the method according to the fourth aspect, said dispersion is cooled to +5°C.

According to an eighth aspect of the present invention, the method according to the aspects from fourth to seventh, comprises at least the further steps of controlling at least a pH, a jelling temperature and a PA titre; as well as a bacteric load and stability of said ALA-gel vehicle.

According to a ninth aspect of the present invention, the method according to any of said aspects from fourth to eighth, comprises the further step of individually evaluating an influence of the amount of said poloxamer 407 (Lutrol 127), either per se or in a mixture with at least another of said poloxamers having the structural formula according to the first aspect, on the jelling temperature of the ALA-gel vehicle in a presence of a preset range of excipients and/or active principles.

According to a tenth aspect of the present invention, the method according to the aspects from fourth to ninth, provides a composition or preparation with an ALA-gel vehicle according to the aspects from first to third, for oncological applications and also for dermo-aesthetical applications, in particular for treating acne skin photo-ageing disorders.

To demonstrate the efficiency of the inventive vehicle, even with a reduced ALA concentration, several evaluations have been performed by comparing the fluorescence read-out of cutaneous or skin zones incubated by the subject ALA-gel (5%) and "traditional" or prior art vehicles (PEG Unguentum-10%).

To also demonstrate the advantageous characteristic of the incubation times of a preparation for dermatologic applications made by using the ALA-gel vehicle according to the present invention, evaluations have been moreover carried out by comparing the fluorescence read-out in skin zones incubated by the inventive ALA-gel (5%) and "traditional" or prior art vehicles (PEG-Unguentum-10%).

Finally, to demonstrate the no need of any further different steps and complex occlusions, evaluations or tests have been moreover carried out by comparing the fluorescence read-out of skin zones incubated by the subject ALA-gel (5%).

More specifically, the efficiency of the inventive ALA-gel vehicle has been evaluated, as above stated, by detecting its fluorescence.

In other words, the improved efficiency of the inventive ALA-gel vehicle in photodynamic applications has been clearly proved, by demonstrating that the PpIX concentration endpoint in tissues is achieved in a shorter time and without a need of performing bandage occlusions.

To clearly understand the evaluation criteria, it should be pointed out that delta-aminolevulinic acid (ALA) which is the metabolite of the eme biosynthesis, is highly selective for neoplastic and dysplastic tissues.

Under physiologic conditions, a formation of ALA in cells is controlled by the ALA-synthase enzyme; as ALA is applied from outside a cell, the enzymatic adjusting mechanism is temporarily bypassed and also exogenous ALA is metabolized to Protoporphyrin IX (PpIX).

As known, fluorescence is a secondary light emission after proper stimulation of a molecule or atom by an absorption of electromagnetic energy, thereby assuming a higher energy status.

Such an emission occurs as a molecule returns to an energy base level; typically such a process occurs very quickly (a few tens of nanosecond for PpIX) thereby fluorescence stops as the exciting light is removed.

This emission has always a wavelength which is greater (and accordingly an energy which is lower) that the excitation and, in the PpIX case, the response peak or maximum value occurs at about 635 nm, as shown for example in Figure 1.

Said PpIX shows a strong excitation peak or maximum value at 405 nm (blue-violet); thus this wavelength is preferably used; the skin is observed or imaged through a barring optical filter allowing the fluorescence emission to pass through but fully locking the excitation at 405 nm.

Since the fluorescence intensity is directly related to the contents of PpIX in the tissues, it constitutes an effective method for evaluating "in vivo" the inventive vehicle efficiency in favoring the ALA conveying and absorption.

In particular, for the above mentioned evaluation have been used the following :

### Materials and methods

Pairs of lesions suitable for a photodynamic treatment with symmetry and similitude characteristics as to a clinic aspect, and being arranged at easily accessible zones, were identified or found.

On the first element of said pair a control means was applied consisting of an ALA dispersion (Sigma-Aldrich) in an ointment form including PEG (MOST) with an ALA concentration corresponding to 10%; then an occlusive medication by using a transparent film (Tegaderm-3M) was performed.

On the second element of the above pair, the ALA-gel vehicle according to the present invention was applied without any occlusive medication.

A reference colored "sight" was then preset to evaluate (based on a visual comparison) if the fluorescence achieves the target endpoint.

Every 15 minutes, each lesion of the pair was illuminated by a monochromatic source at 405 nm (SP-405N - AlphaStrumenti), and the fluorescence detected was evaluated with respect to the reference colored "sight".

Moreover, the period of time (in minutes) required to achieve the fluorescence endpoint for each said lesion was calculated.

### Results

The fluorescence detecting campaign involved 23 persons (age 35 to 72) for a total of 35 measurements.

The detected data are shown in the diagrams of Figure 2, the X axis showing a progressive number of the experimental tests, and the Y axis showing the time required for achieving the fluorescence endpoint.

### Conclusions

The detected data show the improved efficiency of the ALA-gel vehicle compared to a reference vehicle, even operating:
- in an absence of any occlusions
- with an active principle concentration of 5% instead of 10%.

On average, a 30% reduction of the contact time for achieving the same fluorescence read-out was detected.

From the above it should be apparent that the invention fully achieves the intended aim and objects.

Although the invention has been disclosed with reference to specific embodiments thereof, it should be apparent that these embodiments have been hereinabove disclosed only by way of an example and not a limitation, and that they are susceptible to several modifications and variations all coming within the scope of the invention, as defined by the accompanying claims.

For example, all the above mentioned and claimed number values relating to the component amounts should be considered as preceded by the term "about".

### Biblioaraphic References

[DOUG-98] Photodynamic Therapy J. Dougherty et al. J Natl Cancer Inst 1998;90:889-905
[LANC-04] The present and future role of photodynamic therapy in cancer treatment S. Brown et al. Lancet Oncol 2004 5: 497-508
[PHAGE-08] Molecular Effects of Photodynamic Therapy for Photoaging J S. Orringer, et al. Arch Dermatol. 2008;144(10):1296-1302
[FLUO-02] M Monti et al. - comunicazione interna ICH X-2002
[GUID-07] Guidelines on the use of photodynamic therapy for non-melanoma skin cancer: An international consensus Lasse R. Braathen et al. JAm Acad Dermatol 2007;56:125-43.

## Claims

1. An ALA-gel vehicle for favoring an absorption and conveying of an active principle for a topic use in particular in a photodynamic therapy with delta-aminolevulinic acid in dermatologic applications, **characterized in that** said vehicle comprises a clear aqueous solution jelling at a temperature from 20 to 37°C, preferably from 25 to 27°C, of the following components: poloxamer 407 (Lutrol 127) as a vehicle-gel either per se or in a mixture with one or more of the poloxamers having the following structural formula: 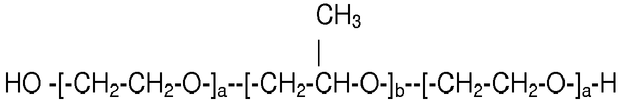 said vehicle comprising moreover potassium sorbate and sodium benzoate as preserving agents, 5-amminolevunic chloridrate as a precursor agent for providing a target photodynamic reaction, and purified water.

2. An ALA-gel vehicle, according to claim 1, **characterized in that** said ALA-gel has the following formulation in weight percent: poloxamer 407 (Lutrol 127) from 1 to 40%, preferably 17%; potassium sorbate preferably 0.20%; sodium benzoate preferably 0.50%; 5-aminolevulinic chloridrate preferably from 1 to 30%, more preferably 1.00%, and purified water q.s. to 100%.

3. An ALA-gel vehicle, **characterized in that** said vehicle is separately preserved and that the pharmaceutical preparation for topical use is extemporaneously constituted before use by adding said ALA-gel vehicle to the other components of said pharmaceutical preparation or composition.

4. A method for preparing the ALA-gel vehicle according to any of the preceding claims 1 to 3, **characterized in that** said method comprises at least the steps of:
cooling a preset amount of purified water;
dissolving under stirring potassium sorbate or sodium benzoate in said purified water;
dispersing 5-aminolevulinic chloridrate and poloxamer 407 (Lutrol 127) and further stirring the dispersion thus obtained; and
cooling said dispersion up to a full dissolving of said poloxamer 407 (Lutrol 127) thereby providing a clear solution jelling in a temperature range of substantially 25-27°C.

5. A method according to claim 4, **characterized in that** said purified water preset amount is cooled to +5°C.

6. A method according to claim 4, **characterized in that** said dispersion is further stirred for 10-15 minutes.

7. A method according to claim 4, **characterized in that** said dispersion is cooled to +5 °C.

8. A method according to any of the preceding claims 4 to 7, **characterized in that** said method further comprises the steps of controlling at least a pH, a jelling temperature and a P.A. titre, and a bacteric load and stability of said vehicle.

9. A method according to any of the preceding claims 4 to 8, **characterized in that** said method comprises the further step of individually evaluating an influence of the amount of said poloxamer 407 either per se or in a mixture with at least another of said poloxamers having the structural formula according to claim 1, on the jelling temperature of the ALA-gel vehicle in a presence of a preset range of excipients and/or active principles.

10. A method according to any of the preceding claims 4 to 9, providing an ALA-gel vehicle composition or preparation according to any of the preceding claims 1 to 3 for oncologic applications and also dermo-aesthetical applications, in particular for treating acne skin photo-ageing disorders.
